Europäisches Patentamt

European Patent Office

Office européen des brevets

(11) Numéro de publication: **0 210 918**
**A1**

## (12) DEMANDE DE BREVET EUROPEEN

(21) Numéro de dépôt: 86401626.6

(22) Date de dépôt: 21.07.86

(51) Int. Cl.⁴: **A61F 13/12 , A42B 1/08 , A42B 3/02**

(30) Priorité: 24.07.85 FR 8511322

(43) Date de publication de la demande:
04.02.87 Bulletin 87/06

(84) Etats contractants désignés:
DE GB IT

(71) Demandeur: COMMISSARIAT A L'ENERGIE
ATOMIQUE Etablissement de Caractère
Scientifique Technique et Industriel
31/33, rue de la Fédération
F-75015 Paris(FR)

(72) Inventeur: Bouyssi, Jean-François
122, Boulevard Murat
F-75016 Paris(FR)
Inventeur: Massard, Thierry
155, Boulevard Brune
F-75014 Paris(FR)
Inventeur: Vienney, Jean
13, rue de la Chartreuse
F-91510 Lardy(FR)

(74) Mandataire: Mongrédien, André et al
c/o BREVATOME 25, rue de Ponthieu
F-75008 Paris(FR)

(54) Calotte de protection pour boîte crânienne et son procédé de fabrication.

(57) La présente invention a pour objet une calotte de protection pour boîte crânienne ainsi qu'un procédé de fabrication de cette calotte de protection.

On prend d'abord une empreinte (4) de la boîte crânienne (2), ce qui permet de réaliser une réplique (12) du crâne ; sur cette dernière, on dépose successivement un film thermostable (14), puis une première couche en tissu de carbone, une deuxième couche de fibre aramide et une troisième couche de tissu de carbone ; ces trois couches forment la calotte de protection (16) après polymérisation dans un four (18) et élimination de la réplique du crâne et du film thermostable ; la calotte peut éventuellement être percée de trous (20).

Application à la protection du cortex cérébral.

_a_

## CALOTTE DE PROTECTION POUR BOITE CRANIENNE ET SON PROCEDE DE FABRICATION

La présente invention se rapporte au domaine des protections pour cortex cérébral et a plus spécialement pour objet une protection plus légère et plus agréable à porter que les dispositifs de l'art antérieur.

Certaines personnes présentent des défaillances de la boîte crânienne, soit par malformation congénitale, soit encore à la suite d'un traumatisme grave. Le cortex cérébral n'est plus alors protégé naturellement par les os crâniens. En général, ces personnes sont contraintes de porter un casque de type moto, ce qui assure une protection complète, ou encore un casque de cycliste en cuir qui n'assure qu'une protection minimale. Dans tous les cas, il s'agit d'un objet relativement lourd et voyant. Le patient est contraint de porter ce casque en permanence, ce qui lui interdit un grand nombre d'activités sportives ou même sociales. En particulier, dans le cas d'enfants ou d'adolescents, le caractère très voyant de la protection ne contribue pas à un épanouissement normal de la personnalité (complexe d'invalide).

La présente invention a justement pour but d'éliminer ces inconvénients en proposant une calotte de protection pour boîte crânienne plus légère et plus facile à porter que les casques utilisés à l'heure actuelle.

Selon la principale caractéristique de la calotte de protection objet de l'invention, celle-ci étant destinée à recouvrir au moins une partie d'une boîte crânienne, cette calotte comprend, de l'intérieur vers l'extérieur :

-une première couche en tissu de carbone,

-une deuxième couche en tissu de fibre aramide, et

-une troisième couche en tissu de carbone.

De préférence, l'une au moins de ces couches, et si possible les trois, est imprégnée d'une résine, cette dernière étant de préférence une résine à haute élongation. On peut encore prévoir une pluralité de trous dont chacun traverse les trois couches de la calotte.

Selon une autre caractéristique de l'invention, l'épaisseur de cette calotte est inférieure à 1 mm et de préférence inférieure à 0,5 mm, cette épaisseur étant généralement comprise entre 0,3 et 0,5 mm.

L'invention a également pour objet un procédé de réalisation d'une calotte de protection pour boîte crânienne. Selon la principale caractéristique de ce procédé, celui-ci comporte les étapes suivantes consistant à :

a) réaliser une réplique de la boîte crânienne,

b) déposer successivement, sur la réplique de la boîte crânienne, une première couche en tissu de carbone, une deuxième couche en fibre aramide, et une troisième couche en tissu de carbone,

c) soumettre l'ensemble à un traitement de polymérisation, et

d) éliminer la réplique de la boîte crânienne.

Selon une autre caractéristique de ce procédé, la réalisation de la réplique de la boîte crânienne comprend les étapes suivantes consistant à :

1) prendre une empreinte de la boîte crânienne afin d'obtenir un moule en un premier matériau, ce moule présentant une cavité,

2) couler un deuxième matériau dans cette cavité,

3) laisser durcir le deuxième matériau afin d'obtenir une masse solide, et

4) extraire cette masse solide qui constitue la réplique de la boîte crânienne.

Eventuellement, le procédé comporte une étape supplémentaire effectuée après l'étape a et avant l'étape b, consistant à placer un film thermostable sur la réplique de la boîte crânienne, ce film thermostable étant ultérieurement retiré après l'étape d. Il peut également être avantageux de prévoir une autre étape, effectuée avant l'étape b, consistant à déposer un agent démoulant avant de déposer la première couche.

Selon une autre caractéristique de ce procédé, le traitement de polymérisation est effectué en autoclave à la température de 125°C pendant 24 heures.

L'élimination de la réplique de la boîte crânienne peut se faire par tous moyens connus, par exemple par des moyens mécaniques ou par dissolution dans un solvant approprié.

On peut encore prévoir une étape supplémentaire, effectuée après l'étape d, consistant à percer des trous dont chacun traverse les trois couches. Enfin, on peut encore prévoir une autre étape, effectuée après l'étape d, consistant à usiner la calotte de protection une fois terminée afin de l'adapter aux dimensions de la zone du crâne que l'on veut protéger.

L'invention apparaîtra mieux à la lecture de la description qui va suivre, donnée à titre d'exemple purement illustratif et nullement limitatif, en référence aux dessins annexés, dans lesquels :

-les figures 1a à 1g sont des vues - schématiques illustrant les différentes étapes du procédé de réalisation de la calotte de protection objet de la présente invention,

-la figure 2 est une vue schématique en coupe montrant la structure d'une telle calotte placée sur une boîte crânienne, et

-la figure 3 est une vue schématique montrant comment on peut placer une perruque afin de camoufler cette calotte de protection.

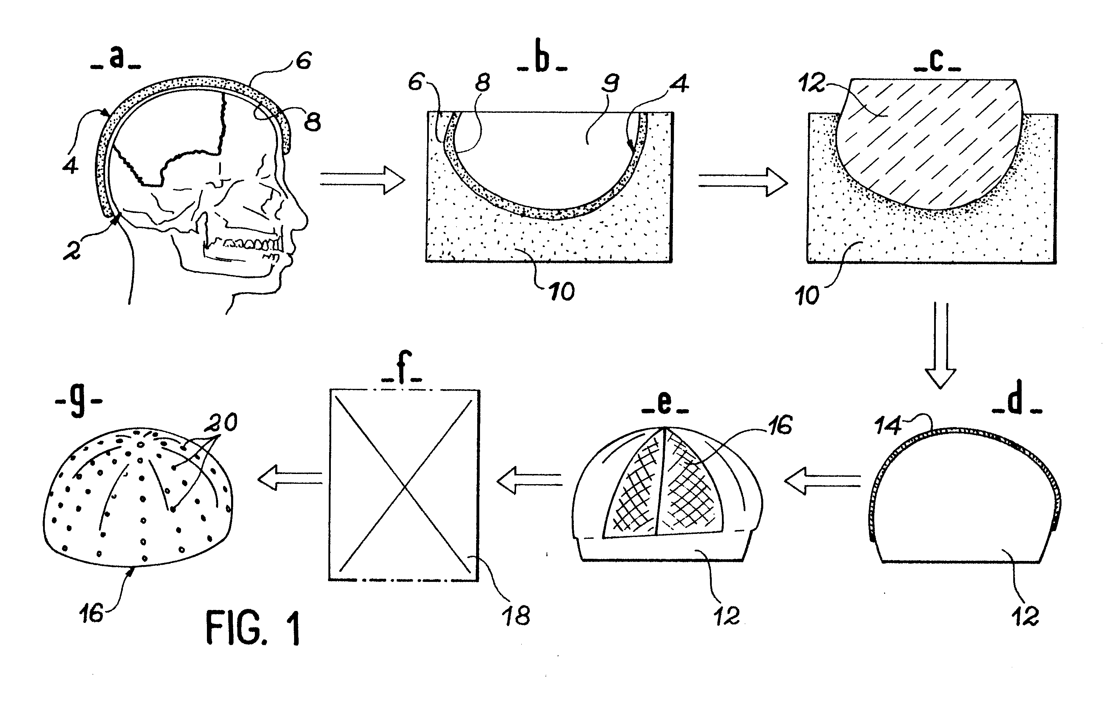

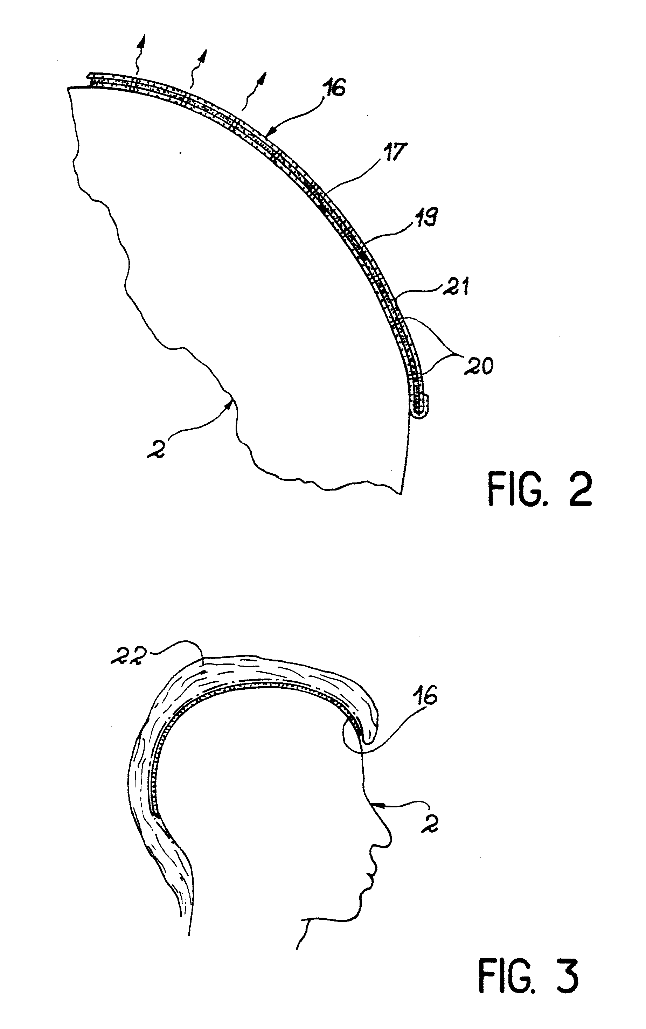

La réalisation d'une telle calotte est illustrée aux figures 1a à 1g. Sur la figure 1a, on voit que l'on commence par réaliser une empreinte de la boîte crânienne 2 en réalisant, en tout matériau approprié, une calotte provisoire 4 présentant une face externe 6 et une face interne 8. La figure 1b montre que l'on place ensuite la calotte provisoire 4 dans une masse 10 d'un premier matériau, la face externe 6 de la calotte provisoire 4 étant en contact avec ce matériau tandis que la face interne 8 est tournée vers le haut, définissant ainsi une cavité 9. Pour réaliser la réplique du crâne, on coule dans la cavité 9 une masse 12 d'un matériau approprié, par exemple du plâtre (figure 1c), et on laisse durcir ce deuxième matériau afin d'obtenir une masse solide 12. Cette masse solide est ensuite extraite du moule 10 et constitue une réplique exacte de la boîte crânienne 2. La figure 1d montre que l'opération suivante consiste à placer un film thermostable, par exemple un film vinylique 14, sur la réplique 12 de la boîte crânienne. Le rôle de ce film thermostable sera expliqué plus loin dans la suite de la présente description. L'étape suivante, illustrée à la figure 1e, consiste à placer successivement les trois couches mentionnées ci-dessus et qui sont destinées à réaliser la calotte de protection 16. La constitution de cette calotte sera expliquée ci-dessous en référence à la figure 2. Une fois que les trois couches formant la calotte 16 sont déposées, on place l'ensemble dans un four 18 (figure 1f) où il subit un traitement de polymérisation, qui peut être un traitement en autoclave à la température de 125°C pendant 24 heures. Après cuisson, l'ensemble est extrait du four et on élimine la réplique 12 de la boîte crânienne, soit par une opération mécanique, soit par dissolution dans un solvant. On retire ensuite le film thermostable et, si on le désire, on perce un certain nombre de trous 20 dans la calotte 16 (figure 1g). La présence des trous 20 permet les échanges thermiques naturels de la peau et évite une sudation trop importante.

Il est à noter que la calotte de protection 16 subit un léger retrait dû au traitement de polymérisation dans le four 18. C'est pour tenir compte de ce retrait qu'on place le film thermostable 14 illustré à la figure 1d afin qu'après retrait, les dimensions de la calotte de protection 16 correspondent exactement à celles de la boîte crânienne.

La structure de cette calotte apparaît mieux sur la figure 2 où l'on voit qu'elle se compose, de l'intérieur vers l'extérieur, de trois couches, à savoir :

- une première couche 17 en tissu de carbone,

-une deuxième couche 19 en tissu de fibre aramide, et

-une troisième couche 21 en tissu de carbone.

De préférence, les trois couches sont imprégnées d'une résine à très haute élongation.

On peut utiliser à cette fin une résine époxy à laquelle est incorporé un ester et ayant les caractéristiques suivantes à une température de 20°C :

| | Module d'Young (MPa) | Contrainte à la rupture (MPa) | Allongement à la rupture (%) |
|---|---|---|---|
| Traction | 20 | 5,6 | 60 |
| Compression | 85 | 49,0 | 65 |

Une telle résine peut être obtenue par exemple en mélangeant 42,9% en poids du produit HYCAR CTBN de la société GOODRICH importé en France par la société POLYPLASTIQUE, 25,9% en poids du produit EPIKOTE de la société SHELL CHIMIE, 30,2% d'anhydride dodécanyl succinique et 1% d'octoate d'étain. Cette résine, qui est liquide avant polymérisation, se polymérise par un traitement de 24 heures à une température de l'ordre de 120 à 125°C. On imprègne les trois couches constituant la calotte lorsque la résine est encore liquide, puis on met la calotte pendant 24 heures dans un four à 120 ou 125°C afin de polymériser la résine.

A titre d'essai, on a réalisé une série de protections de ce type, dans lesquelles les couches 17 et 21 étaient en tissu de carbone TORAY T300 1 000 filaments tandis que la deuxième couche 19 était en tissu de fibre aramide KEVLAR satin. Chacune des trois couches était imprégnée de la résine à très haute élongation mentionnée ci-dessus. Le carbone donne la rigidité et la résistance de la protection. Le KEVLAR donne une bonne résistance à l'impact et un grand pouvoir amortisseur. Quant à la résine haute élongation utilisée, c'est une résine époxy qui permet d'obtenir des déformations de flexion très grandes, limitant ainsi les risques de détérioration de la protection lorsque le patient la met ou l'enlève.

On voit encore sur la figure 2 que la calotte 16 est percée d'un certain nombre de trous 20 afin de permettre les échanges thermiques entre la peau du crâne et l'air ambiant et d'éviter une sudation trop importante.

La calotte de protection objet de l'invention présente de nombreux avantages dont le principal est qu'elle est légère et agréable à porter. En effet, les calottes qui ont été réalisées ont des épaisseurs variant de 3 à 5/10e de millimètre et un poids variant de 30 à 60 grammes environ. Cette calotte épouse parfaitement la forme de la boîte crânienne et se porte directement sur la peau grâce à sa souplesse. De plus, les matériaux qui la composent donnent, comme on vient de le voir, de très hautes performances mécaniques. D'autre part, comme on peut le voir sur la figure 3, une perruque 22 peut être implantée directement sur la calotte 16, ce qui rend cette dernière totalement invisible et élimine tous les complexes qui pourraient affecter un individu obligé de porter cette protection. La forme de cette calotte, ainsi que sa très grande légèreté, la rend supportable pendant de longues périodes d'autant plus que la présence des trous 20 permet les échanges thermiques naturels de la peau et évite une sudation trop importante.

**Revendications**

1. Calotte de protection destinée à recouvrir au moins une partie d'une boîte crânienne (2), caractérisée en ce qu'elle comprend, de l'intérieur vers l'extérieur :

-une première couche (17) en tissu de carbone,

-une deuxième couche (19) en tissu de fibre aramide,

et

-une troisième couche (21) en tissu de carbone.

2. Calotte de protection selon la revendication 1, caractérisée en ce que l'une au moins des trois couches (17, 19, 21) est imprégnée d'une résine.

3. Calotte de protection selon la revendication 2, caractérisée en ce que la résine est une résine à haute élongation.

4. Calotte de protection selon l'une quelconque des revendication 1 à 3, caractérisée en ce qu'elle comporte une pluralité de trous (20) dont chacun traverse les trois couches (17, 19, 21).

5. Calotte de protection selon l'une quelconque des revendications 1 à 4, caractérisée en ce que son épaisseur est inférieure ou égale à 1 mm.

6. Calotte de protection selon la revendication 5, caractérisée en ce que son épaisseur est inférieure ou égale à 0,5 mm.

7. Calotte de protection selon la revendication 6, caractérisée en ce que son épaisseur est comprise entre 0,3 et 0,5 mm.

8. Procédé de réalisation d'une calotte de protection pour boîte crânienne, caractérisé en ce qu'il comprend les étapes suivantes consistant à :

a) réaliser une réplique (12) de la boîte crânienne (2),

b) déposer successivement, sur la réplique - (12) de la boîte crânienne (2), une première couche (17) en tissu de carbone, une deuxième couche (19) en fibre aramide et une troisième couche - (21) en tissu de carbone,

c) soumettre l'ensemble à un traitement de polymérisation, et

d) éliminer la réplique (12) de la boîte crânienne.

9. Procédé selon la revendication 8, caractérisé en ce que la réalisation de la réplique (12) de la boîte crânienne (2) comprend les étapes suivantes consistant à :

1) prendre une empreinte de la boîte crânienne (2) afin d'obtenir un moule (10) en un premier matériau et présentant une cavité (9),

2) couler un deuxième matériau dans cette cavité (9),

3) laisser durcir le deuxième matériau, afin d'obtenir une masse solide (12), et

4) extraire cette masse solide qui constitue la réplique (12) de la boîte crânienne (2).

10. Procédé selon l'une quelconque des revendications 8 et 9, caractérisé en ce qu'il comporte une étape supplémentaire, effectuée après l'étape a et avant l'étape b, consistant à placer un film thermostable (14) sur la réplique (12) de la boîte crânienne (2), ce film thermostable (14) étant ensuite retiré après l'étape d.

11. Procédé selon l'une quelconque des revendications 8 à 10, caractérisé en ce qu'il comporte une étape supplémentaire, effectuée avant l'étape b, consistant à déposer un agent démoulant avant de déposer la première couche (17).

12. Procédé selon l'une quelconque des revendications 8 à 11, caractérisé en ce que le traitement de polymérisation est effectué en autoclave à la température de 125°C pendant 24 heures.

13. Procédé selon l'une quelconque des revendications 8 à 12, caractérisé en ce que l'élimination de la réplique (12) de la boîte crânienne (2) se fait par des moyens mécaniques.

14. Procédé selon l'une quelconque des revendications 8 à 12, caractérisé en ce que l'élimination de la réplique (12) de la boîte crânienne (2) se fait par dissolution dans un solvant.

15. Procédé selon l'une quelconque des revendications 8 à 14, caractérisé en ce qu'il comporte une étape supplémentaire, effectuée après l'étape d, consistant à percer des trous (20) dont chacun traverse les trois couches (17, 19, 21).

16. Procédé selon l'une quelconque des revendications 8 à 15, caractérisé en ce qu'il comporte une étape supplémentaire, effectuée après l'étape d, consistant à usiner la calotte de protection (16) pour adapter ses dimensions en fonction de la zone à protéger.

-a-

6
8
4
2

-b-
9
4
8
6
10

-c-
12
12
10

-d-
14
12

-e-
16
12

-f-
18

-g-
20
16

FIG. 1

FIG. 2

FIG. 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | GB-A-   9 114  (T.J.F. DE MARTEL DE JANVILLE) (A.D. 1914) * Page 1, lignes 5-31; figures * | 1,8,9 | A 61 F   13/12 A 42 B    1/08 A 42 B    3/02 |
| | --- | | |
| A | FR-A-2 335 169  (A.L. GALLET) * Page 1,  ligne  35  -  page  2, ligne 3; revendications 1,3 * | 1,2 | |
| | --- | | |
| A | CH-A-  322 226  (R.W.C. DEHESDIN) * Page 1, lignes 7-39 * | 1,2 | |
| | --- | | |
| A | US-A-3 320 619  (A.L. LASTNIK) * Colonne 3, lignes 45-69 * | 1,2 | |
| | ----- | | |

|  |
|---|
| DOMAINES TECHNIQUES RECHERCHES (Int. Cl.4) |
| A 61 F A 42 B |

Le présent rapport de recherche a été établi pour toutes les revendications

| Lieu de la recherche LA HAYE | Date d'achèvement de la recherche 06-11-1986 | Examinateur STEENBAKKER J. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82